# FASCICULE DE BREVET EUROPEEN

(11) **EP 2 986 879 B1**
(45) Date de publication et mention de la délivrance du brevet: **04.03.2020**
(21) Numéro de dépôt: 14723461.1
(22) Date de dépôt: 10.04.2014
(51) Int. Cl.: F16L 37/084, F16L 37/098

(54) **CONNEXION FLUIDIQUE SÉCURISÉE**
SICHERE FLÜSSIGKEITSVERBINDUNG
SECURE FLUID CONNECTION

(30) Priorité: 15.04.2013 FR 1353382
(43) Date de publication de la demande: 24.02.2016
(73) Titulaire: SARTORIUS STEDIM FMT SAS, 13400 Aubagne (FR)
(72) Inventeur: MENDYK, Nicolas, F-13124 Peypin (FR)
(74) Mandataire: Derambure Conseil
(86) Numéro de dépôt international: PCT/FR2014/050865
(87) Numéro de publication internationale: WO 2014/170584

(56) Documents cités:
- EP-A1- 2 224 158
- DE-A1- 3 710 853
- FR-A- 1 487 324
- US-A- 5 649 724
- US-A1- 2006 208 743
- US-A1- 2008 252 071
- US-A1- 2010 019 487

## Description

La présente invention est relative aux dispositifs de connexion fluidique notamment pour raccorder ensemble deux conduites de fluide.

Plus particulièrement, l'invention concerne un dispositif de connexion fluidique pour mettre en relation deux conduites fluidiques, que l'on appelle aussi dispositif de raccord ou de raccordement. Le fluide peut être liquide, gazeux ou pâteux, et la conduite peut être un tuyau ou une conduite interne à un appareil.

Il est connu, notamment à partir du document EP0462971, d'utiliser un dispositif de connexion fluidique pour raccorder une première conduite fluide à une deuxième conduite fluide, comprenant :
- une fiche femelle, fabriquée en matière plastique, et liée à la première conduite fluide,
- une fiche mâle, fabriquée en matière plastique, liée à la deuxième conduite fluide, et destinée à venir s'insérer dans la fiche femelle jusque dans une position finale de couplage, et
- un dispositif de verrouillage primaire déplaçable, ayant une position de verrouillage dans laquelle il empêche les fiches mâle et femelle de quitter la position finale de couplage.

Cependant, dans ce document EP0462971, il n'est prévu aucun moyen pour sécuriser la connexion en vue de pouvoir empêcher une déconnexion non désirée. Le document EP 2 224 158 A1 divulgue un dispositif de connexion fluidique selon le préambule de la revendication 1, avec fiche femelle intégrant un verrou secondaire.

Il s'avère pourtant que, seulement dans certains cas d'utilisation, il peut être avantageux de sécuriser la connexion dans la position effective de raccordement pour éviter toute déconnexion ultérieure non souhaitée.

A cet effet, l'invention propose notamment un dispositif de connexion fluidique du type mentionné ci-dessus, **qui présente les caractéristiques de la revendication 1.**

Grâce à ces dispositions, on peut utiliser dans un premier temps le dispositif de connexion fluidique de manière réversible grâce au dispositif de verrouillage primaire et dans un deuxième temps rendre indémontable ladite connexion grâce au dispositif de verrouillage secondaire amené dans sa position active, de manière non réversible ; en effet, lorsque le verrou secondaire a atteint sa position active, cette dernière ne peut plus être quittée par une manœuvre d'un utilisateur, ce qui rend le raccordement définitif. Dans un environnement médical, spatial ou autre, on peut ainsi sécuriser la connexion, ce qui est avantageux pour pouvoir garantir qu'aucune déconnexion non souhaitée ne puisse intervenir, et ce qui est avantageux pour garantir la non rupture de la chaîne stérile et le maintien permanent des conditions d'asepsie

Dans des modes de réalisation selon l'invention:
- le dispositif de verrouillage secondaire peut empêcher le dispositif de verrouillage primaire de s'écarter de sa position de verrouillage ; ce qui représente une solution particulièrement robuste pour assurer l'indémontabilité ;
- le dispositif de verrouillage primaire est rappelé vers sa position de verrouillage par un moyen de rappel élastique ; de sorte que sa position par défaut est la position de verrouillage ;
- le dispositif de verrouillage primaire forme une pièce distincte de la fiche femelle ; on peut ainsi choisir un matériau différent, notamment en fonction des contraintes de biocompatibilité et des exigences d'élasticité ;

Selon un autre aspect, ledit verrou secondaire est prévu comme une pièce séparée, fournie et amenée séparément des fiches mâle et femelle, pièce que l'on vient clipser au moment du verrouillage définitif. De sorte qu'il est aisé de reconnaître une connexion sécurisée par la présence du verrouillage secondaire, que celui-ci soit réversible ou non.

D'autres caractéristiques et avantages de l'invention apparaîtront au cours de la description suivante, en regard des dessins joints.

Sur les dessins :
- la figure 1 montre un exemple de système dans lequel est utilisé un dispositif de connexion selon l'invention,
- la figure 2 est une vue schématique d'un dispositif de connexion selon un premier mode de réalisation l'invention, représenté à l'état désaccouplé,
- les figures 3 et 3A montrent le dispositif de connexion de la figure 2, représenté à l'état accouplé,
- les figures 4 et 4A montrent le dispositif de connexion de la figure 2, représenté à l'état accouplé et sécurisé,
- les figures 5A-5C montrent plus en détail une variante du dispositif de verrouillage secondaire,
- la figure 6 est une vue schématique d'un dispositif de connexion selon un deuxième mode de réalisation (non conforme à l'invention), représenté à l'état désaccouplé,
- la figure 7 montre le dispositif de connexion de la figure 6, représenté à l'état accouplé,
- la figure 8 montre le dispositif de connexion de la figure 6, représenté à l'état accouplé et sécurisé,
- la figure 9 est une vue schématique d'un dispositif de connexion selon un troisième mode de réalisation (non conforme à l'invention), représenté à l'état désaccouplé,
- la figure 10 montre le dispositif de connexion de la figure 9, représenté à l'état accouplé,
- la figure 11 montre le dispositif de connexion de la figure 9, représenté à l'état accouplé et sécurisé,
- les figures 12 et 13 montrent une variante de réalisation du troisième mode de réalisation.

Sur les différentes figures, les mêmes références désignent des éléments identiques ou similaires.

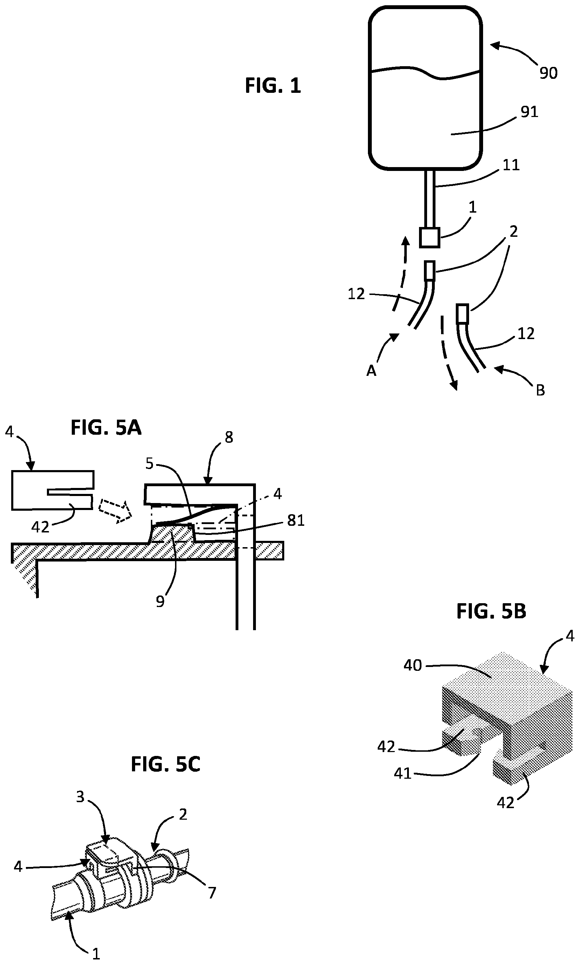

Comme représenté sur la figure 1, une poche réservoir **90** est prévue pour contenir du fluide biopharmaceutique **91 ;** cet usage peut nécessiter des précautions d'asepsie. Dans le contexte de l'invention, on entend par « fluide biopharmaceutique », un fluide issu de la biotechnologie - milieux de cultures, cultures cellulaires, solutions tampon, liquides de nutrition artificielle - ou un fluide pharmaceutique ou plus généralement un fluide destiné à être utilisé dans le domaine médical. Une première conduite fluide **11** met en communication le volume interne de la poche réservoir 90 avec une fiche de connexion **1** (ou raccord). Dans l'exemple illustré, cette fiche est une fiche femelle (raccord femelle) qui est adaptée pour recevoir par complémentarité de forme une fiche mâle **2** sur laquelle est raccordée une deuxième conduite de fluide **12.**

Selon un exemple d'utilisation, on connecte tout d'abord un premier appareil **'A'** ayant une deuxième conduite de fluide 12 et un raccord mâle 2, par exemple pour procéder au remplissage de la poche réservoir souple 90. Une connexion adéquate entre fiches mâle et femelle doit être assurée de façon positive, mais on doit pouvoir ensuite déconnecter la connexion (on peut dire aussi désaccoupler le raccord) une fois que l'opération de remplissage est terminée.

La poche réservoir peut ensuite être utilisée dans un endroit différent et dans un contexte différent le cas échéant. En particulier, ladite poche réservoir **90** peut servir à alimenter un circuit de distribution de fluide ou un appareil de dispense de produit, référencé **'B'** à la figure 1, auquel cas la poche réservoir 90 va se vider dans un circuit utilisateur. Le contexte d'application peut imposer de rendre indissociables la poche souple et au moins une partie du circuit utilisateur, en particulier dans le cas d'éléments à usage unique dont on se débarrasse après l'usage complet, mais aussi dans le cas où on veut garantir que la chaîne de stérilité est bien conservée. À cette fin, le raccord sera mis dans une configuration telle qu'il est impossible de désaccoupler le raccord par une manœuvre, la connexion étant alors sidérée comme assurée de manière définitive.

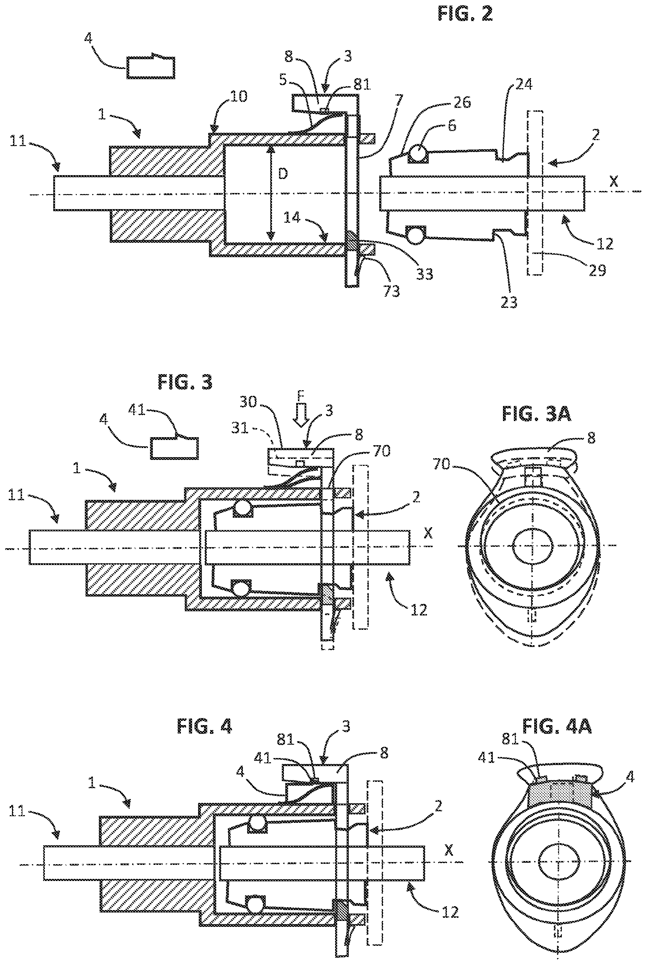

Les figures 2, 3, 3A, 4 et 4A illustrent un premier mode de réalisation de la connexion fluidique, qui comprend une fiche femelle **1,** et une fiche mâle **2,** destinée à venir s'insérer dans la fiche femelle jusque dans une position finale de couplage représentée à la figure 3, selon un mouvement de couplage/découplage parallèle à la direction axiale **X.**

La fiche femelle **1** comprend une portion cylindrique interne de réception **14,** de section circulaire et de diamètre **D,** lequel est légèrement supérieur au diamètre extérieur de la fiche mâle destinée à venir s'insérer dans la fiche femelle. De façon connue, un joint torique **6** positionné dans une gorge de la fiche mâle sert de moyen d'étanchéité entre la fiche mâle la fiche femelle lorsque celles-ci sont en position de couplage comme représenté aux figures 3 et 4.

De plus, il est prévu un dispositif de verrouillage primaire **3** déplaçable, qui se présente comme une plaque **7** disposée transversalement par rapport à l'axe **X,** avec une portion de manœuvre **8** agencée perpendiculairement à ladite plaque. De plus, la plaque **7** comprend un orifice central **70** sensiblement circulaire et de diamètre légèrement supérieur au diamètre de la fiche mâle ; dans l'exemple illustré ici, le dispositif de verrouillage primaire **3** forme une pièce distincte de la fiche femelle **1** proprement dite, ce qui permet de choisir des matériaux plastique différents en fonction des diverses contraintes de compatibilité avec les fluides transportés et les exigences d'élasticité pour la déformation des pièces.

Le dispositif de verrouillage primaire **3,** aussi appelé verrou primaire **3** peut être déplacé entre une position de verrouillage **30** et une position de déverrouillage **31** représentée en traits pointillés sur la figure 3. L'orifice axial 70 du verrou primaire 3 est de diamètre légèrement supérieur au diamètre de la fiche mâle, et dans sa partie inférieure, il est prévu au moins une projection **33** ou une bordure 33 de l'orifice destinée à venir se loger dans une gorge **24** annulaire de la fiche mâle.

Cette projection ou bordure 33 vient par ailleurs en appui sur un épaulement **23** ménagé dans la fiche mâle 2, ce qui permet d'assurer la fonction verrouillage anti retrait.

En effet, lorsque la fiche mâle a été insérée jusque dans la position couplage complète (position finale de couplage) et lorsque le verrou primaire **3** est dans sa position de verrouillage, il empêche les fiches mâle et femelle de quitter la position finale de couplage, car l'épaulement **23** vient buter contre la projection **33** qui se trouve en position haute.

Lors du mouvement d'insertion de la fiche mâle, la partie conique **26** de celle-ci repousse la projection 33 vers le bas de manière à ce que l'orifice axial **70** du verrou primaire 3 se trouve dans une position sensiblement coaxiale à l'axe **X** et autorise la progression du mouvement d'insertion jusqu'à la position finale de couplage. Au cours de ce mouvement, c'est l'ensemble du verrou primaire qui se déplace vers le bas, puis remonte à sa position de verrouillage lorsque les fiches mâle et femelle atteignent la position finale de couplage.

Le verrou primaire est rappelé vers la position de verrouillage au moyen d'un élément de rappel élastique **5** ; dans l'exemple illustré, il s'agit d'une languette élastique 5 qui peut être soit issue du corps cylindrique **10** de la fiche femelle soit issue de la portion de manœuvre **8** du verrou primaire 3. De façon alternative, ce rappel pourrait aussi être procuré par un ressort classique.

Le déverrouillage du verrou primaire reste possible : il suffit d'appuyer sur la portion de manœuvre **8** du verrou primaire **3** (flèche **F**) pour repousser le verrou primaire vers la position de déverrouillage, auquel cas la projection **33** s'efface de l'épaulement **23** et la fiche mâle peut être retirée du logement de la fiche femelle.

Il est à noter qu'en raison de la réaction du joint torique ou d'une force de rappel spécifiquement prévue, la fiche mâle peut avantageusement reculer dès que le verrou primaire la libère ; ceci procure un bon feed-back du déverrouillage pour l'utilisateur ; la position finale couplage étant donc instable, cette disposition permet également de garantir que le verrouillage primaire est effectivement enclenché.

Dans le cadre du présent document, le terme 'position de verrouillage' pour le verrou primaire **3** désigne la position physique représentée aux figures 2 et 4, i.e. ce terme couvre à la fois la position initiale (Fig 2) sans qu'une fiche mâle 2 soit présente et la position de verrouillage effectif avec une fiche mâle insérée et retenue dans la fiche femelle 1 (Fig 4).

Lorsqu'il est nécessaire de rendre définitive la connexion, alors un verrou secondaire **4** est placé dans une position active, comme représenté en Figure 4.

Plus précisément, le verrou secondaire **4** dans l'exemple illustré se présente comme une pièce en forme générale de 'U' ouvert en direction de la portion de manœuvre **8,** l'évidement intérieur de la forme en U étant prévu pour loger l'élément de rappel élastique **5.** On vient placer ce verrou secondaire dans une position active représentée à la figure 4, dans une position où il est interposé entre le corps cylindrique de la fiche femelle et la portion de manoeuvre **8** du verrou primaire 3.

En outre, ce verrou secondaire présente une ou plusieurs formes, dans cet exemple plus précisément deux formes de crochets **41** prévues pour coopérer chacune avec un logement de clipsage correspondant **81.**

De ce fait, une fois que le verrou secondaire est engagé dans sa position active, il est très difficile voire impossible de le retirer, cette position est donc non réversible. La présence de ce verrou secondaire empêche alors de déplacer le verrou primaire depuis sa position de verrouillage vers une position de déverrouillage. Ainsi on obtient un établissement définitif de la connexion.

Il peut être prévu en outre un moyen pour éviter que le verrou primaire ne sorte de son logement ; par exemple une lame élastique **73** agencée sur la plaque **7** diamétralement opposée à la portion de manoeuvre empêche tout retrait vers le haut de du verrou primaire au-delà de la position de verrouillage **30.**

Selon ce premier mode de réalisation, la fiche mâle est particulièrement simple car elle est symétrique de révolution autour de l'axe et contient comme seul élément déformable joint torique. Il peut être prévu optionnellement une collerette **29** qui facilite la préhension et la poussée d'accouplement vers la fiche femelle.

Selon ce premier mode de réalisation, la fiche femelle reçoit le verrou primaire **3** de manière coulissante transversalement à l'axe, on remarque que le verrou primaire est embarqué sur la fiche femelle, il peut se déplacer transversalement entre deux positions mais est prisonnier dans la fiche femelle (il ne peut plus être retiré une fois qu'il y a été inséré) ; la fiche femelle est également prévue pour recevoir le verrou secondaire **4** qui vient s'interposer de manière à bloquer le déplacement du verrou primaire.

Il faut aussi remarquer que le verrou primaire n'est pas en contact avec le fluide qui traverse le raccordement ; en effet, le joint torique 6 est interposé entre l'intérieur des conduites et ledit verrou primaire.

Il faut remarquer que le verrou secondaire 4 ne peut être mis en place correctement que si le verrou primaire 3 est dans sa position de verrouillage (sinon on ne peut pas l'engager car l'espace est insuffisant); par conséquent, si la position de couplage n'a pas été correctement atteinte, l'insertion du verrou secondaire ne sera pas possible et cela donnera à l'utilisateur un feed-back explicite. Cette disposition contribue à assurer la qualité de la connexion.

Dans le cas illustré ici, le verrou secondaire 4 est une pièce séparée qui est amenée au moment où on a besoin de sécuriser de manière définitive la connexion. De manière avantageuse, ce verrou secondaire peut être d'une couleur différente des fiches mâle et femelle, de manière que l'on puisse voir de loin et sans ambiguïté si le verrouillage secondaire est en place ou pas.

Sur les figures 5A-5C, il est représenté plus en détails une variante concernant la fonction de verrouillage secondaire. Un ergot **9** fait saillie de la surface extérieure de la fiche femelle, cet ergot se trouve en dessous de la portion de manœuvre **8.** Le ressort 5 présente une base attachée à la base de ladite portion de manœuvre **8** et une extrémité opposée libre qui porte sur le dessus de l'ergot **9.** Dans l'exemple représenté, le ressort et l'ergot ont sensiblement la même largeur dans la direction circonférentielle.

Le verrou secondaire **4** comprend un corps **40** sensiblement parallélépipédique dont sont issues deux branches latérales **42** flexibles qui sont disposées pour venir s'interfacer avec l'ergot 9. Chacune des branches latérales est munie d'une forme de crochet **41** à son extrémité, configurée pour s'écarter vers l'extérieur au moment de l'insertion du verrou secondaire. Dès lors que les formes en crochet ont franchi la paroi avant 81 de l'ergot, les branches se resserrent et le clipsage est assuré. De plus, il n'est pas possible par une manœuvre utilisateur d'écarter les dites branches, et de fait le clipsage devient non réversible. A partir de cet état, on peut garantir que la connexion ne pourra pas être déconnectée et on peut ainsi garantir la continuité de la chaine stérile si on travaille dans ce genre de conditions. La chaine stérile ne pourrait être rompue que si on casse la connexion ou que si on utilise un outil spécifique pour ôter le verrou secondaire.

Dans une autre variante non représentée, les crochets peuvent présenter des rampes de manière à ce que le clipsage du verrou secondaire soit réversible, aspect qui peut être avantageux dans le cas d'un verrou secondaire formé par une pièce spécifique séparée des fiches.

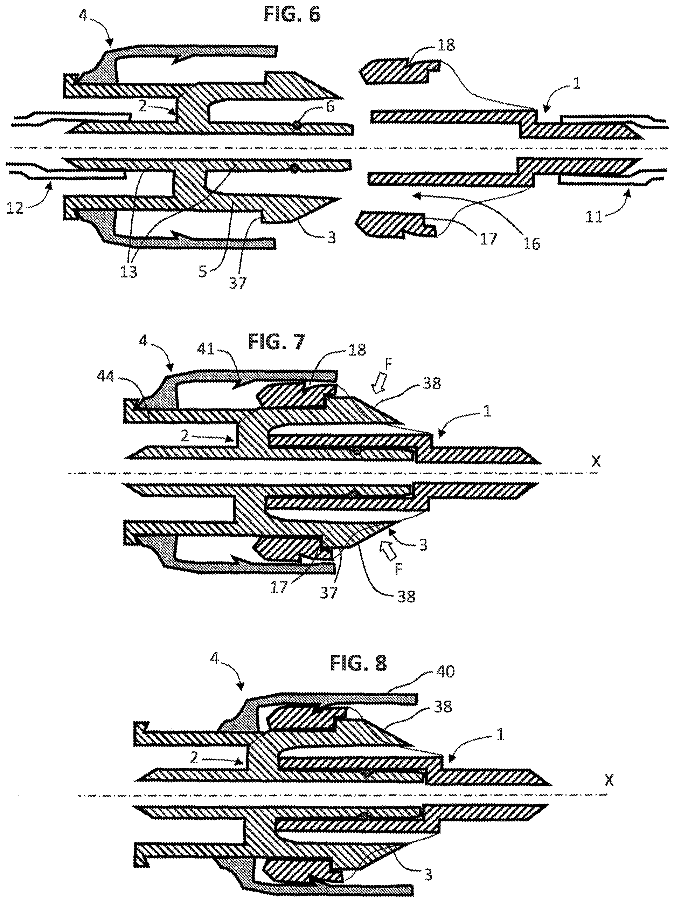

Les figures 6, 7 et 8 illustrent un deuxième mode de réalisation de la connexion fluidique, qui comprend une fiche femelle **1,** et une fiche mâle **2,** comme précédemment. Sur les figures, les fiches mâle et femelle sont présentées coupées dans un plan diamétral qui contient les éléments principaux du couplage et du verrouillage ; sur la majeure partie de la circonférence des pièces, celle-ci peuvent être de manière générale symétriques de révolution autour de l'axe **X,** excepté à l'endroit des éléments principaux de verrouillage.

Avantageusement, la forme générale des fiches mâle femelle peut être similaire aux produits de la série SBL du fournisseur 'Value Plastics', c'est-à-dire présenter une section générale en forme ellipse, avec une dimension radiale plus grande à l'endroit des clips et une dimension radiale plus faible ailleurs.

La fiche mâle **2** comprend une portion centrale cylindrique **13** de révolution configurée d'une part pour être reçu dans la fiche femelle et d'autre part pour recevoir un tube **12** ou tuyau formant la deuxième conduite. De plus la fiche mâle comprend deux clips élastiques 3 diamétralement opposés qui forment le dispositif de verrouillage primaire de la connexion. Les branches 5 portant les clips de verrouillage sont déformables élastiquement selon une direction radiale ; elles peuvent fléchir contrainte en direction de l'axe mais reviennent en l'absence de sollicitation externe vers une position de repos plus écartée de l'axe.

Enfin, la fiche mâle porte également le verrou secondaire **4** qui se trouve dans une position d'attente (aussi dite 'inactive'), telle que représentée sur les figures 5 et 6, une portée arrière **44** étant prévu pour maintenir et guider ledit verrou secondaire selon un mouvement longitudinal. Le verrou secondaire 4 est réalisé en matière plastique relativement souple et est disposé de façon sensiblement annulaire autour de la portion arrière de la fiche mâle.

De son côté, la fiche femelle **1** comprend des logements **16** s'étendant axialement et aptes à recevoir les clips diamétraux 3 susmentionnés. Sur la partie arrière de chaque logement, se situe un épaulement **17** sur lequel viendra buter une forme en saillie **37** du clip. La première conduite fluide 11 est insérée sur l'arrière de la fiche femelle **1.**

Sur la figure 7, les fiches mâle et femelle ont atteint la position finale de couplage, pour laquelle la forme en saillie **37** est verrouillée sur l'épaulement **17.** Dans cette configuration, il est encore possible de désaccoupler la connexion en pressant les deux clips diamétraux **3** l'un en direction de l'autre, comme illustré sur les flèches **F.** Tant que le verrou secondaire 4 reste dans sa position inactive, alors on peut accoupler et désaccoupler les fiches mâle et femelle à l'aide du verrouillage primaire.

Lorsque l'on veut rendre la connexion définitive, il faut alors déplacer longitudinalement le verrou secondaire 4 de sa position d'attente vers sa position active telle que représentée à la figure 8. Dans cette position, l'accès aux zones de poussée **38** sur les clips du verrouillage primaire est très difficile voire impossible avec les doigts, ce qui permet d'éviter toute déconnexion non désirée du raccordement fluide et garantir la non rupture de la chaine stérile.

De plus, le verrou secondaire est équipé de formes de harpons **41** qui viennent se loger dans une rainure extérieure **18** de la fiche femelle de manière à rendre impossible le retour en arrière de la position active vers la position inactive. On obtient alors un verrouillage définitif de la connexion.

Il faut remarquer que dans ce second mode de réalisation, on pourrait positionner le verrou secondaire 4 dès le début dans sa position active, auquel cas on obtiendrait une connexion indémontable dès le premier accouplement.

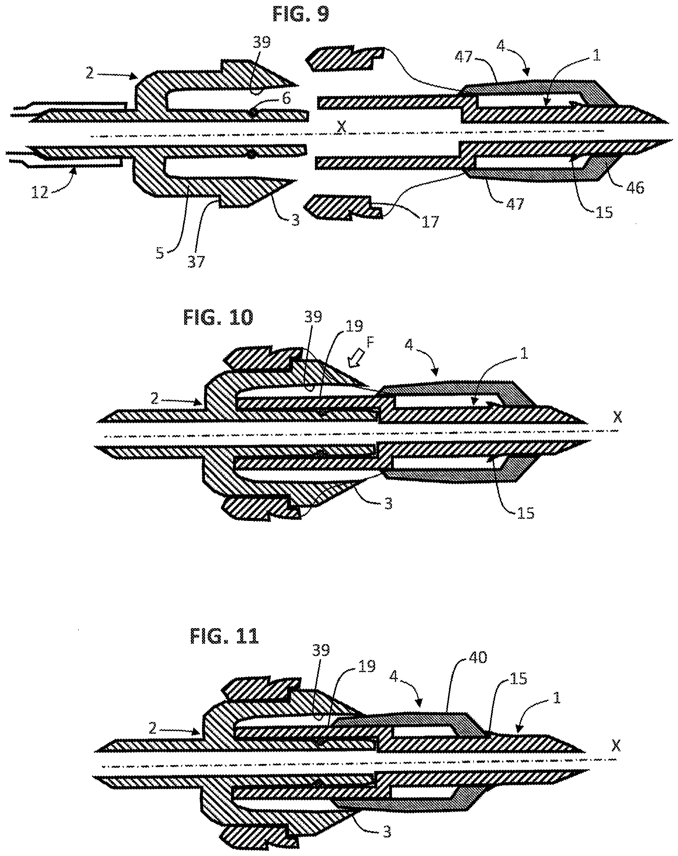

Les figures 9, 10 et 11 illustrent un troisième mode de réalisation de la connexion fluidique, qui comprend une fiche femelle **1,** et une fiche mâle **2,** comme précédemment.

Tout comme dans le deuxième mode, le dispositif de verrouillage primaire se présente sous la forme de deux clips diamétralement opposées agencées sur la fiche mâle. Le fonctionnement du dispositif de verrouillage primaire est tout à fait similaire à ce qui a été décrit pour le deuxième mode de réalisation.

En revanche, s'agissant du verrou secondaire **4,** celui-ci est agencé du côté de la prise femelle **1,** et comporte une portion annulaire arrière **46** qui entoure une zone tubulaire arrière de la fiche femelle ; il présente aussi au moins deux pattes **47** s'étendant axialement, lesdites pattes étant prévues pour venir s'interposer entre respectivement la portion intérieure avant **39** de chaque clip **3** et le corps cylindrique extérieur **19** de la fiche femelle de manière à rendre impossible le déplacement des clips depuis leur position de verrouillage. L'espace libre nécessaire aux mouvements de déverrouillage est alors occupé par les pattes **47** qui empêchent tout mouvement de déverrouillage des clips **3.**

De plus, la portion tubulaire arrière de la fiche femelle comprend des formes **15** (ici des formes de harpons) pouvant coopérer avec le verrou secondaire pour le retenir dans la position active une fois que celui-ci y a été amené, comme illustré à la figure 11. En effet, dès lors que le verrou secondaire 4 a été déplacé vers la fiche femelle suffisamment, les formes 15 sont dépassées et jouent un rôle de blocage pour empêcher tout mouvement du verrou secondaire vers la droite.

Dans cette configuration, il est à noter que l'encombrement radial peut être diminué, mais l'emprise axiale est un peu plus importante ; dans le deuxième mode, inversement, la dimension axiale de la connexion sécurisée est optimisée, avec une dimension radiale un peu plus importante.

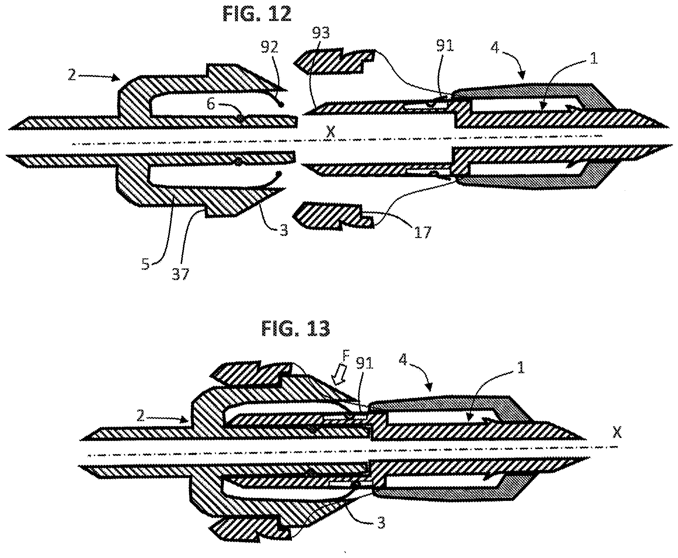

Comme représenté aux figures 12 et 13, il est prévu une variante dont le but est d'empêcher d'amener le verrou secondaire 4 dans sa position active tant que la connexion n'est pas dans la position finale de couplage et tant que le verrou primaire n'est pas dans sa position de verrouillage.

Plus précisément, sur la fiche femelle 1 sont disposées des lances déformables **91,** qui dans leur position de repos empêchent le mouvement du verrou secondaire vers sa position active (cf Fig 12). Chacune de ces lances déformables 91 est destinée à être déplacée par une anse élastique **92** agencée en vis-à-vis sur la portion de tête du clip 3.

Lorsque la fiche mâle vient s'insérer dans la fiche femelle, l'anse élastique 92 porte d'abord sur le chanfrein **93** de la bordure avant de la fiche femelle puis vient presser la lance déformable 91 en direction radiale vers l'intérieur ; de ce fait, celle-ci s'efface alors vers l'intérieur et autorise le verrou secondaire 4 à se déplacer vers la gauche (cf Fig 13).

Donc le verrou secondaire **4** ne peut quitter sa position inactive que à la condition que la position finale de couplage soit atteinte et que le verrou primaire soit dans la position verrouillée.

Les autres caractéristiques et fonctionnalités sont identiques ou similaires à ce qui a été décrit pour le troisième mode de réalisation ci-dessus.

Dans des cas d'application typique de l'invention, le diamètre de la conduite fluide est typiquement compris entre 1 et 15 mm. L'encombrement radial les fiches mâle et femelle peut être compris entre 10 mm et 30 mm.

Avantageusement, les pièces décrites sont réalisées en matière plastique ; certaines d'entre elles peuvent être transparentes de manière à ce qu'on puisse réaliser un contrôle visuel de ce qui se passe dans la canalisation fluide ; de plus le verrou secondaire peut être d'une couleur particulièrement voyante pour que sa présence puisse être vérifiée facilement (cf Fig. 5C).

## Revendications

1. Dispositif de connexion fluidique (10) pour raccorder une première conduite fluide (11) à une deuxième conduite fluide (12), le dispositif comprenant :
- une fiche femelle (1) fabriquée en matière plastique, et liée à la première conduite fluide,
- une fiche mâle (2) fabriquée en matière plastique, liée à la deuxième conduite fluide, et destinée à venir s'insérer dans la fiche femelle jusque dans une position finale de couplage, selon un mouvement de couplage/découplage parallèle à une direction axiale (X),
- un dispositif de verrouillage primaire (3) déplaçable transversalement entre deux positions, agencé sur la fiche femelle et formant une pièce distincte de la fiche femelle et distincte de la fiche mâle, ayant une position de verrouillage (30) dans laquelle il empêche les fiches mâle et femelle de quitter la position finale de couplage, le dispositif de verrouillage primaire (3) étant embarqué sur et prisonnier dans la fiche femelle (1) de manière coulissante transversalement à la direction axiale (X), le dispositif de verrouillage primaire (3) se présentant comme une plaque (7) disposée transversalement par rapport à la direction axiale (X), avec une portion de manœuvre (8) agencée perpendiculairement à ladite plaque, la plaque (7) comprenant un orifice central (70) axial sensiblement circulaire et de diamètre légèrement supérieur au diamètre de la fiche mâle (2), au moins une projection (33) ou une bordure (33) étant prévue dans une partie inférieure de l'orifice (70) et destinée à venir se loger dans une gorge annulaire de la fiche mâle, la projection (33) ou bordure (33) venant en appui sur un épaulement (23) ménagé dans la fiche mâle (2), ce qui permet d'assurer une fonction verrouillage anti retrait,
- un verrou secondaire (4) déplaçable, ayant une position active adaptée pour empêcher la manœuvre du dispositif de verrouillage primaire (3) à partir de sa position de verrouillage, le verrou secondaire étant une pièce séparée, pouvant venir s'interposer, dans sa position active, de manière à bloquer le déplacement du dispositif de verrouillage primaire, le verrou secondaire étant configuré pour être clipsé de manière non réversible dans sa position active, moyennant quoi le dispositif de connexion fluidique établit un raccordement définitif et indémontable des première et deuxième conduites, de manière à garantir que la chaîne de stérilité est bien conservée,
dans lequel le verrou secondaire (4) ne peut être amené en position active que si le dispositif de verrouillage primaire (3) est dans sa position de verrouillage, le verrou secondaire (4) empêchant le dispositif de verrouillage primaire (3) de s'écarter de sa position de verrouillage,
**caractérisé en ce que** le verrou secondaire (4) est une pièce pouvant être fournie et amenée séparément des fiches mâle et femelle (1, 2), de sorte que la fiche femelle (1) et la fiche mâle (2) puissent être assemblées entre elles dans leur position finale de couplage en présence du dispositif de verrouillage primaire (3) prisonnier dans la fiche femelle (1) et en l'absence du verrou secondaire (4), et **en ce que** la fiche femelle (1) est prévue pour recevoir, sélectivement après l'obtention de la position finale de couplage et après l'obtention de la position de verrouillage (30), le verrou secondaire (4).

2. Dispositif selon la revendication 1, dans lequel le dispositif de verrouillage primaire (3) présente une portion de manœuvre (8) pour repousser le dispositif de verrouillage primaire vers une position de déverrouillage, auquel cas ladite projection (33) s'efface de l'épaulement (23) et la fiche mâle (2) est déplacée selon un mouvement de découplage parallèle à la direction axiale (X), et dans lequel le verrou secondaire (4) empêche l'accès à la manœuvre du dispositif de verrouillage primaire (3) lorsque le verrou secondaire (4) est dans la position active, en étant interposé entre un corps cylindrique de la fiche femelle (1) et la portion de manœuvre (8) du dispositif de verrouillage primaire (3) qui est agencé perpendiculairement à ladite plaque (7).

3. Dispositif selon l'une des revendications 1 à 2, dans lequel le dispositif de verrouillage primaire (3) est rappelé vers sa position de verrouillage (30) par un moyen de rappel élastique (5).

4. Dispositif selon l'une des revendications 1 à 3, dans lequel l'encombrement radial pour chacune parmi la fiche femelle (1) et la fiche mâle (2) est compris entre 10 mm et 30 mm.

5. Utilisation du dispositif de connexion fluidique (10) selon l'une quelconque des revendications 1 à 4, dans laquelle le dispositif de connexion fluidique sert à raccorder ensemble deux conduites pour un fluide liquide dans le domaine médical.

6. Utilisation du dispositif de connexion fluidique (10) selon l'une quelconque des revendications 1 à 4, dans laquelle le dispositif de connexion fluidique sert à raccorder ensemble deux conduites pour un fluide gazeux apte à être utilisé dans le domaine médical.

7. Utilisation du dispositif de connexion fluidique (10) selon l'une quelconque des revendications 1 à 4, dans laquelle le dispositif de connexion fluidique sert à raccorder ensemble deux conduites pour un fluide pâteux apte à être utilisé dans le domaine médical.

## Patentansprüche

1. Fluidverbindungsvorrichtung (10) zum Anschließen einer ersten Fluidleitung (11) an eine zweite Fluidleitung (12), wobei die Vorrichtung aufweist:
- eine aus einem Kunststoffmaterial hergestellte Steckbuchse (1), die mit der ersten Fluidleitung verbunden ist,
- einen aus einem Kunststoffmaterial hergestellten Stecker (2), der mit der zweiten Fluidleitung verbunden ist und konfiguriert ist, gemäß einer zu einer axialen Richtung (X) parallelen Kopplung/Entkopplung-Bewegung in die Steckbuchse bis zu einer Kopplungsendposition eingeführt zu werden,
- eine zwischen zwei Positionen transversal verschiebbare primäre Verriegelungsvorrichtung (3), die an der Steckbuchse angeordnet ist und ein zu der Steckbuchse separates und zu dem Stecker separates Stück bildet und eine Verriegelungsposition (30) hat, in der sie verhindert, dass der Stecker und die Steckbuchse die Kopplungsendposition verlassen, wobei die primäre Verriegelungsvorrichtung (3) auf eine transversal zur axialen Richtung (X) gleitende Weise an der Steckbuchse (1) gebordet und darin gefangen ist, wobei sich die primäre Verriegelungsvorrichtung (3) als eine transversal bezüglich der axialen Richtung (X) angeordnete Platte (7) präsentiert, mit einem senkrecht zu der Platte angeordneten Betätigungsabschnitt (8), wobei die Platte (7) eine im Wesentlichen kreisförmige axiale zentrale Öffnung (70) aufweist, deren Durchmesser geringfügig größer als der Durchmesser des Steckers (2) ist, wobei mindestens ein Vorsprung (33) oder eine Leiste (33) in einem unteren Teil der Öffnung (70) vorgesehen ist und dazu bestimmt ist, in einer ringförmigen Auskehlung des Steckers aufgenommen zu werden, wobei der Vorsprung (33) oder die Leiste (33) an einer in dem Stecker (2) ausgesparten Schulter (23) zum Anschlag kommt, was erlaubt, eine gegen Zurückziehen gesicherte Verriegelungsfunktion zu gewährleisten,
- einen verschiebbaren sekundären Riegel (4), der eine geeignete aktive Position hat, um das Betätigen der primären Verriegelungsvorrichtung (3) aus ihrer Verriegelungsposition zu verhindern, wobei der sekundäre Riegel ein separates Stück ist, das in seiner aktiven Position in Eingriff kommen kann, um die Verschiebung der primären Verriegelungsvorrichtung zu blockieren, wobei der sekundäre Riegel konfiguriert ist, auf eine nichtreversible Weise in seiner aktiven Position eingerastet zu werden, wodurch die Fluidverbindungsvorrichtung einen endgültigen und nichtlösbaren Zusammenschluss der ersten und zweiten Leitung herstellt, um so zu garantieren, dass die Sterilitätskette gut gewahrt bleibt,
in welcher der sekundäre Riegel (4) nur dann in die aktive Position gebracht werden kann, wenn die primäre Verriegelungsvorrichtung (3) in ihrer Verriegelungsposition ist, wobei der sekundäre Riegel (4) verhindert, dass sich die primäre Verriegelungsvorrichtung (3) aus ihrer Verriegelungsposition entfernt,
**dadurch gekennzeichnet, dass** der sekundäre Riegel (4) ein Stück ist, das separat zu der Steckbuchse und dem Stecker (1, 2) geliefert und bereitgestellt werden kann, so dass die Steckbuchse (1) und der Stecker (2) in Anwesenheit der in der Steckbuchse (1) gefangenen primären Verriegelungsvorrichtung (3) und in Abwesenheit des sekundären Riegels (4) in ihre Kopplungsendposition zusammengefügt werden können, und dadurch, dass die Steckbuchse (1) vorgesehen ist, selektiv nach Erreichen der Kopplungsendposition und nach Erreichen der Verriegelungsposition (30) den sekundären Riegel (4) zu empfangen.

2. Vorrichtung nach Anspruch 1, in welcher die primäre Verriegelungsvorrichtung (3) einen Betätigungsabschnitt (8) aufweist, um die primäre Verriegelungsvorrichtung hin zu einer Entriegelungsposition zu stoßen, in welchem Fall der Vorsprung (33) sich von der Schulter (23) löst und der Stecker (2) sich gemäß einer Entkopplungsbewegung, die zur axialen Richtung (X) parallel ist, verschiebt, und in welcher der sekundäre Riegel (4) den Zugriff auf das Betätigen der primären Verriegelungsvorrichtung (3) verhindert, wenn der sekundäre Riegel (4) in der aktiven Position ist, in der er zwischen einem zylindrischen Körper der Steckbuchse (1) und dem Betätigungsabschnitt (8) der primären Verriegelungsvorrichtung (3) angeordnet ist, die senkrecht zu der Platte (7) ist.

3. Vorrichtung nach einem der Ansprüche 1 bis 2, in welcher die primäre Verriegelungsvorrichtung (3) mit Hilfe einer elastischen Rückstelleinrichtung (5) in ihre Verriegelungsposition (30) zurückgestellt wird.

4. Vorrichtung nach einem der Ansprüche 1 bis 3, in welcher die radiale Abmessung sowohl der Steckbuchse (1) als auch des Steckers (2) zwischen 10mm und 30mm ist.

5. Verwendung der Fluidverbindungsvorrichtung (10) nach einem der Ansprüche 1 bis 4, in welcher die Fluidverbindungsvorrichtung dazu dient, zwei Leitungen für ein auf dem medizinischen Gebiet verwendbares flüssiges Fluid zusammenzuschließen.

6. Verwendung der Fluidverbindungsvorrichtung (10) nach einem der Ansprüche 1 bis 4, in welcher die Fluidverbindungsvorrichtung dazu dient, zwei Leitungen für ein auf dem medizinischen Gebiet verwendbares gasförmiges Fluid zusammenzuschließen.

7. Verwendung der Fluidverbindungsvorrichtung (10) nach einem der Ansprüche 1 bis 4, in welcher die Fluidverbindungsvorrichtung dazu dient, zwei Leitungen für ein auf dem medizinischen Gebiet verwendbares pastenartiges Fluid zusammenzuschließen.

## Claims

1. Fluid connection device (10) for connecting a first fluid conduit (11) to a second fluid conduit (12), comprising:
- a female plug (1), made of plastic and joined to the first fluid conduit,
- a male plug (2), made of plastic, joined to the second fluid conduit, and intended for insertion into the female plug into a final coupling position, according to a coupling/decoupling movement parallel to an axial direction (X),
- a movable primary locking device (3) able to move transversely between two positions, arranged on the female plug and forming a part separate from the female plug and separate from the male plug, having a locked position (30) in which it prevents the male and female plugs from being released from the final coupling position, the primary locking device (3) being embedded and trapped on the female plug in a sliding manner transversely to the axial direction (X), the primary locking device (3) being provided in the form of a plate (7) arranged transversely to the axial direction (X), with a manipulating portion (8) arranged perpendicular to said plate, the plate (7) comprising a substantially circular central hole (70) of a slightly larger diameter than the diameter of the male plug (2), at least one projection (33) or rim (33) being provided in a lower portion of the hole (70) and intended to be received in an annular groove of the male plug, the projection or rim (33) bearing against a shoulder (23) formed on the male plug (2), which ensures a retraction-preventing locking function,
- a movable secondary lock (4) having an active position adapted to prevent the primary locking device (3) from being moved from its locked position, the secondary lock being a separate part able to be interposed, in its active position, in a manner that prevents movement of the primary locking device, the secondary lock being configured to be irreversibly clipped into place in its active position, whereby the fluid connection device establishes a permanent, non-separable connection of the first and second conduits, for ensuring that the chain of sterility remains intact,
wherein the secondary lock (4) can only be placed in its active position if the primary locking device (3) is in its locked position, the secondary lock (4) preventing the primary locking device (3) from moving out of its locked position;
**characterized in that** the secondary lock (4) is a piece able to be supplied and moved separately from the male and female plugs (1, 2), such that the female plug (1) and the male plug (2) can be assembled together in their final coupling position when the primary locking device (3), trapped in the female plug, is present and in the absence of the secondary lock (4), and
**in that** the female plug (1) is adapted to receive the secondary lock (4), selectively after obtaining the final coupling position and after obtaining the locked position (30) .

2. Device according to claim 1, wherein the primary locking device (3) has a manipulating portion (8) to push the primary locking device back to an unlocked position, in which case the projection (33) clears the shoulder (23) and the male plug (2) is displaced according to a decoupling movement parallel to the axial direction (X),
and wherein the secondary lock (4) prevents operative access to the primary locking device (3) when the secondary lock (4) is in the active position, where it is interposed between the cylindrical body of the female plug (1) and the manipulating portion (8) of the primary locking device (3) that is perpendicular to said plate (7).

3. Device according to one of claims 1 to 2, wherein the primary locking device (3) is biased toward the locked position by means of an elastic biasing element (5).

4. Device according to one of claims 1 to 3, wherein the radial footprint of each amongst the female plug (1) and the male plug (2) is between 10 mm and 30 mm.

5. Use of the fluid connection device (10) according to one of claims 1 to 4, wherein the fluid connection device is used for connecting together two conduits for a liquid fluid in the medical field.

6. Use of the fluid connection device (10) according to one of claims 1 to 4, wherein the fluid connection device is used for connecting together two conduits for a gaseous fluid suitable to be used in the medical field.

7. Use of the fluid connection device (10) according to one of claims 1 to 4, wherein the fluid connection device is used for connecting together two conduits for a pasty fluid suitable to be used in the medical field.
